(19) Europäisches Patentamt / European Patent Office / Office européen des brevets

(11) **EP 4 007 614 B1**

## (12) EUROPEAN PATENT SPECIFICATION

(45) Date of publication and mention
of the grant of the patent:
**27.05.2026 Bulletin 2026/22**

(21) Application number: **20746239.1**

(22) Date of filing: **31.07.2020**

(51) International Patent Classification (IPC):
**A61L 27/18** (2006.01)     **A61L 27/24** (2006.01)
**A61L 27/54** (2006.01)     **A61L 27/56** (2006.01)

(52) Cooperative Patent Classification (CPC):
(C-Sets available)
**A61L 27/54; A61L 27/18; A61L 27/24; A61L 27/56;**
A61L 2300/252; A61L 2300/426; A61L 2300/62;
A61L 2430/02; A61L 2430/12          (Cont.)

(86) International application number:
**PCT/EP2020/071734**

(87) International publication number:
**WO 2021/019096 (04.02.2021 Gazette 2021/05)**

(54) **COMPLEMENT ACTIVE FRAGMENT-LOADED THREE-DIMENSIONAL BIOMATERIAL FOR DENTAL AND/OR OTHER TISSUE REGENERATION**

MIT AKTIVEM KOMPLEMENTFRAGMENT BELADENES DREIDIMENSIONALES BIOMATERIAL FÜR DIE REGENERATION VON ZAHNGEWEBE UND/ODER ANDEREM GEWEBE

BIOMATÉRIAU ACTIF TRIDIMENSIONNEL COMPLÉMENTAIRE CHARGÉ EN FRAGMENTS POUR RÉGÉNÉRATION DENTAIRE ET/OU AUTRE RÉGÉNÉRATION DE TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2019 EP 19189635**

(43) Date of publication of application:
**08.06.2022 Bulletin 2022/23**

(73) Proprietors:
• **Septodont ou Septodont SAS ou Specialites Septodont**
**94100 Saint Maur des Fossés (FR)**
• **Université d'Aix Marseille**
**13284 Marseille Cedex 07 (FR)**
• **Centre national de la recherche scientifique**
**75016 Paris (FR)**

(72) Inventors:
• **ABOUT, Imad**
**13190 Allauch (FR)**
• **RICHARD, Gilles**
**91560 Crosne (FR)**

• **BERGMANN, Madison**
**54000 Nancy (FR)**
• **JEANNEAU, Charlotte**
**13009 Marseille (FR)**
• **DJOUDI, Mounir**
**35740 Pacé (FR)**

(74) Representative: **Icosa**
**83 avenue Denfert-Rochereau**
**75014 Paris (FR)**

(56) References cited:
**WO-A1-2013/098331**

• **RAMBHIA KUNAL J ET AL: "Controlled drug release for tissue engineering", JOURNAL OF CONTROLLED RELEASE, ELSEVIER, AMSTERDAM, NL, vol. 219, 29 August 2015 (2015-08-29), pages 119 - 128, XP029303657, ISSN: 0168-3659, DOI: 10.1016/ J.JCONREL.2015.08.049**

- **CHUNRONG YANG ET AL: "Fabrication of Multiple Layered Scaffolds with Controlled Porous Micro-Architecture", MATERIALS RESEARCH, vol. 20, no. 2, 16 January 2017 (2017-01-16), BR, pages 349 - 355, XP055663041, ISSN: 1516-1439, DOI: 10.1590/1980-5373-mr-2016-0703**

(52) Cooperative Patent Classification (CPC): (Cont.)

C-Sets
**A61L 27/18, C08L 67/04**

**Description**

**FIELD OF INVENTION**

The present invention relates to the field of tissue repair and regeneration, especially of dental tissue. The present invention provides a three-dimensional biomaterial comprising particles encapsulating at least one complement active fragment dispersed therein. The invention also refers to a process of manufacturing of said biomaterial. The biomaterial of the invention can advantageously be used in the regeneration of dental pulp, dentin, gingival, periodontal, as well as in the regeneration of bones tissues.

**BACKGROUND OF INVENTION**

Dental diseases, including caries and periodontal diseases, are a major oral health problem. Therefore, the development of reliable and affordable therapeutic strategies for treating dental diseases is an area of active investigations.

In the field of oral health, and also other fields of medicine, therapeutic strategies enabling to regenerate tissues and not only restoring them is in full growth. Especially, researches are conducted to provide method of regeneration of dental pulp, dentin, bones or gingival tissues.

Tissue regeneration requires three major elements: stem cells, scaffolds and activation signals.

Even if most tissues have an important ability of regeneration in case of small lesions, tissue regeneration is limited when the lesion reaches a critical size. In such cases, it is necessary to stimulate the formation of new vessels and the recruitment of stem cells.

Collagen is present in the pulp, dentin and bones tissues and plays a vital role in the angiogenesis process and in bone regeneration. However, the induction of bone regeneration is a slow process.

Several studies have shown that providing stem cells in damaged tissues enables stimulating their regeneration. Nevertheless, stem cell therapy has many drawbacks such as related costs, but also technical and practical concerns.

It has been demonstrated that the complement system, which belongs to innate immunity, is involved in physiological processes of regeneration. The activation of the complement system leads to the production of bioactive fragments. It has especially been demonstrated that the release of such bioactive fragments of the complement, for example C5a, induces the recruitment of progenitor cells of pulp, which is essential in the initiation of the regeneration process (Chmilewsky F. et al, J. Dent. Res., 2013, 92(6), pp.532-539). It has also been demonstrated that these fragments induce the migration of mesenchymal stem cells whose activation is essential in bone regeneration (Ignatius A. et al., J. Trauma., 2011, 71, pp. 952-960). Therefore, if a bioactive fragment of the complement is integrated in a material, it might attract bone and pulp stem cells and all other stem cells expressing the receptors of these complement fragments.

WO2013/098331 discloses a combination of complement proteins C3a/C4a, growth factors, cytokines, antibacterial/antiviral factors; stem cell stimulating factors and chemotactic factors, for the treatment of conditions requiring tissue repair and regeneration. WO2013/098331 states that disclosed combination may be used in combination with a biomaterial such as collagen and can impregnate a scaffold, leading to a restorative material, useful in the treatment of pit caries and pulp inflammation. This type of use within a scaffold is nevertheless not detailed nor exemplified and there is nothing in WO2013/098331 evidencing that complement proteins C3a/C4a can remain stable in such scaffold, nor if it can be efficiently released from the scaffold, especially under an active form. Further, it is not shown if such material may provide a sustained effect overtime.

The present invention aims at providing a biomaterial capable of inducing and accelerating tissue regeneration, especially of dentin-pulp tissues. Complement active fragments were selected as bioactive components to induce the recruitment of stem cells to initiate the regeneration. The difficulty was to provide a biomaterial having a biocompatible and/or resorbable matrix suitable to enable cell progression, adapted to include a complement active fragment and to release it under an active form, sustainedly overtime.

Complement active fragments, such as C5a, have a complex three-dimensional structure. For example, C5a is an ~11 kDa, 74 amino acid glycoprotein having four anti-parallel alpha helices connected by peptide loops, stabilized by three critical disulphide linkages (Manthey et al., Int. J. Biochem. Cell Biol., 2009, 41, 2114-2117). The integrity of the three-dimensional structure is critical to ensure the biological function of the complement active fragments. When the three-dimensional structure of a complement active fragment is affected, it loses its biological function.

Besides, complement active fragments, such as C5a, have a stability dependent to temperature conditions. For example, manufacturers recommend a storage of C5a at a low temperature, between -20°C and -70°C and ensure a stability after reconstitution (i.e. in aqueous solutions) of 3 months when stored between -20°C and -70°C, but only of 1 month if stored between 2°C and 8°C.

In view of the sensibility of complement active fragments, their handling and incorporation in a biomaterial may be critical and may result in the release of an inactive form due to three-dimensional structure impairments or in the loose of a part of the incorporated complement active fragments by degradation. Moreover, even if the three-dimensional structure is not affected by the incorporation into the biomaterial and if manufacturing conditions do not lead to thermal degradation, the efficiency of the release of complement active fragments under active forms is not foreseeable.

The biomaterial of the invention is a three-dimensional biomaterial in which are dispersed particles encapsulating complement components, complement active fragments, or combinations thereof. As evidenced hereafter, the biomaterial of the invention enables a controlled release of the complement component or complement active fragment, with a retained activity despite their intermediate encapsulation and dispersion in the three-dimensional matrix.

The recruitment of pulp progenitor cells is especially guided by a gradient of complement active fragment C5a. Therefore, in order to accelerate dentin-pulp regeneration, it is proposed to provide a biomaterial according to the invention comprising C5a as complement active fragment, that (1) releases C5a to form a C5a gradient from the site of injury to progenitor cells and (2) in which the three-dimensional matrix is suitable to facilitate cells progression.

The biomaterial of the invention presents the advantage to render possible a complete regeneration of the pulp volume. It enables to avoid an important reduction of the pulp volume, contrary to what is observed with previous materials used in direct pulp capping or in pulpotomy procedures. This is particularly advantageous because reduction of the pulp volume leads to a decreased pulp regeneration capacity in case of secondary caries.

## SUMMARY

This invention thus relates to a biomaterial comprising:

- a three-dimensional matrix made of at least one biocompatible and/or resorbable polymer, and
- particles encapsulating a complement component, a complement active fragment, or a combination thereof; said particles being dispersed in the three-dimensional matrix,
- wherein said encapsulating particles are polymeric microspheres and the polymer of the microspheres is selected from biocompatible and/or biodegradable polyesters; preferably selected from polycaprolactone (PCL), poly(lactic acid) (PLA), poly(glycolic acid) (PGA), polyhydroxybutyrate (PHB), poly(3-hydroxy valerate), poly(ethylene succinate) (PESu), poly(butylene succinate) (PBSu) and any combinations or copolymers thereof; preferably the polymer microspheres are made of poly(lactic-co-glycolic acid) (PLGA).

According to one embodiment, the biocompatible and/or resorbable polymer is selected from polysaccharides, proteins and any combinations or copolymers thereof; preferably is selected from collagen, chitosan, alginate and any combinations or copolymers thereof; more preferably is collagen.

According to one embodiment, the complement component and complement active fragment are selected from complement component C1, complement component C2, complement component C3, complement component C4, complement component C5, complement active fragments thereof such as C5a or C3a and any combinations thereof; preferably complement component C5 or C3; more preferably complement active fragment C5a or C3a.

According to one embodiment, the biomaterial comprises PLGA microspheres encapsulating complement active fragment C5a, said microspheres being dispersed in a collagen three-dimensional matrix.

According to one embodiment, the biomaterial further comprises a second phase made of a three-dimensional matrix made of at least one biocompatible and/or resorbable polymer which is free of particles encapsulating a complement component, a complement active fragment, or a combination thereof.

According to one embodiment, the three-dimensional matrix of the biomaterial is porous. According to one embodiment, the three-dimensional matrix of the biomaterial is a hydrogel.

The invention further relates to a process for manufacturing a biomaterial according to the invention, said process comprising contacting particles encapsulating a complement component, a complement active fragment, or a combination thereof, with at least one biocompatible and/or resorbable polymer to form a three-dimensional matrix made of the at least one biocompatible and/or resorbable polymer in which the particles are dispersed, wherein said particles are polymeric microspheres and the polymer of the microspheres is selected from polycaprolactone (PCL), poly(lactic acid) (PLA), poly(glycolic acid) (PGA), polyhydroxybutyrate (PHB), poly(3-hydroxy valerate), poly(ethylene succinate) (PESu), poly(butylene succinate) (PBSu) and any combinations or copolymers thereof.

According to one embodiment, the particles encapsulating a complement component, a complement active fragment, or a combination thereof, are contacted with the biocompatible and/or resorbable polymer by impregnation in a hydrogel formed with the biocompatible and/or resorbable polymer. In this case, the process may further comprise a step of water removal. According to one embodiment, the particles encapsulating a complement component, a complement active fragment, or a combination thereof, are contacted with the biocompatible and/or resorbable polymer by impregnation in a hydrogel prepared by dissolution of said polymer in an aqueous solution followed by homogenization. In this case, the process may further comprise a step of water removal to provide the biomaterial under the form of a sponge.

The invention also relates to the biomaterial according to the invention for use in tissue repair and/or regeneration. In one embodiment, the tissue is selected from dental pulp, dentin, periodontal and gingival tissues. In another embodiment, the tissue is bone.

**DEFINITIONS**

In the present invention, the following terms have the following meanings:

**"Biocompatible"** refers to a material eliciting little or no toxic response in a given organism, and/or which is able to integrate with a particular cell type or tissue.

**"Biomaterial"** refers to any material that is suitable for interaction with biological systems and/or living tissues, preferably for a medical purpose - either a therapeutic (treat, augment, repair or replace a tissue function of the body) or a diagnostic one.

**"Complement active fragment"** refers to an active Complement molecule that is present in the plasma and can play a significant function in the regeneration process. An example of complement active fragment is C5a.

**"Complement component"** refers to a Complement molecule that is present in the plasma and requires an activation step to be active. An example of complement component is C5.

**"Copolymer"** refers to a polymer derived from at least two different monomeric species. Copolymers can be alternating, periodic, statistical, random or block copolymers.

**"Dental tissue"** refers to the various tissues of teeth or of direct environment of teeth. For example, dental tissue refers to dental pulp, radicular pulp, periapical tissue, periodontal tissue, cementum, dentin, tertiary dentin, reactionary dentin, reparative dentin, dentinal tubules, or any tissues associated with dental pulp containing any of fibroblasts, odontoblasts, endothelial cells or immune cells including, histiocytes, macrophage, granulocytes, mast cells or plasma cells.

**"Dentin"** refers to a calcified tissue of the body, and along with enamel, cementum, and dental pulp is one of the four major components of teeth. Dentin is usually covered by enamel on the crown and cementum on the root and surrounds the entire pulp.

**"Dental pulp"** refers to a soft living tissue inside a tooth and is confined within the coronal region and root canals of the tooth.

**"Encapsulating particles"** refers to particles that include inside one or more active compound.

**"Hydrogel"** refers to a two- or multi-component system consisting of a three-dimensional network of polymer chains and water that fills the space between. In one embodiment, the hydrogel can be prepared by dissolution of the polymer in an aqueous solution followed by homogenization.

**"Microspheres"** refers to small spherical particles, with diameters in the micrometer range (typically 1 $\mu$m to 1000 $\mu$m (1 mm)). A **"polymeric microsphere"** refers to a microsphere wherein the particle is made of polymer.

**"Monosaccharide"** refers to polyhydroxy aldehydes or polyhydroxy ketones, comprising at least carbon atoms, and which are not hydrolysable. Non limitative examples of monosaccharides are trioses (glyceraldehyde, dihydroxyacetone); tetroses (erythrose, threose, erythrulose); pentoses (desoxyribose, ribose, arabinose, xylose, lyxose, ribulose, xylulose); hexoses (allose, altrose, galactose, glucose, gulose, idose, mannose, talose, fructose, psicose, sorbose, tagatose); desoxy-hexoses (fucose, rhamnose); heptoses (sedoheptulose, mannoheptulose); nonoses (neuraminic acid or sialic acid).

**"Polyester"** refers to a category of polymers that contain an ester functional group in their main chain. Examples of polyesters include poly(lactic acid) (PLA), poly(glycolic acid) (PGA) and polycaprolactone (PCL).

**"Polymer"** refers to a large organic molecule formed by the repetition of a structural unit.

**"Polysaccharide"** refers to a polymeric carbohydrate molecule composed of long chains of monosaccharide units bound together by glycosidic linkages; which may be linear or branched. Examples of polysaccharides include chitosan and alginate.

**"Porous"** refers to a material comprising pores at the surface and inside of said material.

**"Progenitor cell",** e.g., a stem cell, refers to a relatively undifferentiated cell capable of self-renewal through mitotic cell division and also capable of differentiating into more specialized cell types. As is known in the art, stem cells include adult stem cells which are pluripotent, i.e., capable of differentiating into almost all cell types including types from all three germ layers, multipotent, i.e., capable of differentiating into several cell types of a closely related family of cells, or unipotent, i.e., capable of differentiating into only one type of cell but distinguished from non-stem cells by the ability to self-renew by mitosis.

**"Protein"** refers to a functional entity formed of one or more peptides or polypeptides, and optionally of non-polypeptides cofactors. A "peptide" refers to a linear polymer of amino acids of less than 50 amino acids linked together by peptide bonds; and a "polypeptide" refers to a linear polymer of at least 50 amino acids linked together by peptide bonds.

**"Resorbable"** refers to a material that progressively disappears in a biologic environment.

**"Subject"** refers to a mammal, preferably a human. In the sense of the present invention, a subject may be a patient, i.e. a person receiving medical attention, undergoing or having underwent a medical treatment, or monitored for the development of a disease.

**"Three-dimensional matrix",** that may also be referred to as three-dimensional scaffold or three-dimensional framework refers to structure in 3 dimensions. The three-dimensional matrix may be for example under the form of a sponge of a gel. Especially in the present invention, a three-dimensional matrix provides a suitable microenvironment for the incorporation of cells to regenerate damaged tissues.

**"Tissue regeneration"** refers to both regeneration of tissue with recourse to exactly the type of tissue to be regenerated, in the sense of an increase in the mass of the tissue, as well as the production of new tissue starting from a different type of tissue or cell than that to be produced. For example, the term "dental tissue regeneration" refers to the process by which new dental tissues is produced from progenitor cells, such as stem cells, e.g., dental pulp stem cells.

**"Tissue repair"** refers to healing of a tissue after injury or infection by the production of connective tissue. For example, repair of dental tissues includes the use of pulp capping materials that are used to fill the space created in the tooth and pulp following removal of decay or caused by a trauma. Skin repair occurs after injury healing by producing a scar tissue.

**"Treatment"** refers to therapeutic treatment wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for the targeted disorder if, after receiving a therapeutic amount of the biomaterial according to the methods of the present invention, the patient shows observable and/or measurable reduction or absence of the damage in the tissue; and/or relief to some extent, one or more of the symptoms associated with the tissue damage; and improvement in quality of life issues. The above parameters for assessing successful treatment and improvement in the disease are readily measurable by routine procedures familiar to a physician.

## DETAILED DESCRIPTION

### Biomaterial

The present invention thus relates to a biomaterial carrying at least one complement component, complement active fragment, or combination thereof. In one embodiment, the biomaterial of the invention is a three-dimensional matrix in which a complement component, complement active fragment, or combination thereof, is dispersed. In one embodiment, the biomaterial is a three-dimensional matrix in which a complement component, complement active fragment, or combination thereof, encapsulated in a vehicle is dispersed.

In one embodiment, the biomaterial of the invention comprises at least one complement component, complement active fragment or combination thereof. In one embodiment, the complement component is selected from complement component C1, complement component C2, complement component C3, complement component C4, complement component C5 and any combinations thereof. In a preferred embodiment, the complement component is complement component C5. In one embodiment, the complement active fragment is selected from C5a, C3a and any combinations thereof. In a preferred embodiment, the complement active fragment is C5a. Complement active fragment C5a is particularly advantageous since it plays an important role in the regeneration of the dentin-pulp complex. In another embodiment, the complement active fragment is C3a.

The complement component, complement active fragment or combination thereof present in the biomaterial of the invention is encapsulated in a vehicle. Encapsulation of the complement component and/or complement active fragment enables to provide for enhanced stability thereof and prolonged delivery.

Said encapsulation vehicles are polymeric microspheres.

Polymeric microspheres can be produced using naturally occurring or synthetic polymers and are usually particulate systems in the size range of 0.1 to 500 $\mu$m. Preferably, the average diameter of the encapsulating microsphere ranges from 1 to 100 $\mu$m; preferably from 5 $\mu$m to 50 $\mu$m; preferably from 10 $\mu$m to 30 $\mu$m; more preferably is about 25 $\mu$m. According to one embodiment, the average diameter of the encapsulating microsphere ranges from 10 to 90 $\mu$m; preferably from 10 to 80 $\mu$m, from 10 to 70 $\mu$m, from 10 to 60 $\mu$m, from 10 to 50 $\mu$m or from 10 to 40 $\mu$m. According to one embodiment, the average diameter of the encapsulating microsphere is about 1, 5, 10, 15, 20, 25, 30, 35, 40, 45, 50, 55, 60, 65, 70, 75, 80, 85, 90, 95 or 100 $\mu$m.

The polymer of the encapsulating microspheres is selected from biocompatible and/or biodegradable polyesters; preferably selected from polycaprolactone (PCL), poly(lactic acid) (PLA), poly(glycolic acid) (PGA), polyhydroxybutyrate (PHB), poly(3-hydroxy valerate), poly(ethylene succinate) (PESu), poly(butylene succinate) (PBSu) and any combinations or copolymers thereof such as for example poly(lactic-co-glycolic acid) (PLGA). In one embodiment, the polymer is selected from polycaprolactone (PCL), poly(lactic acid) (PLA), poly(glycolic acid) (PGA) and any combinations or copolymers thereof such as for example poly(lactic-co-glycolic acid) (PLGA). In a specific embodiment, polymeric microspheres are made of PLGA.

Encapsulation can be performed for example using a water/oil single emulsion method or a water/oil/water double emulsion method. In a preferred embodiment, encapsulation is performed using a water/oil/water double emulsion method.

In one embodiment, the biomaterial of the invention comprises a matrix, preferably a three-dimensional matrix. The matrix may enable for cells to adhere and guide the process of tissue repair or regeneration in vivo

In one embodiment, the matrix is made of at least one biocompatible and/or resorbable polymer. In one embodiment, the biocompatible and/or resorbable polymer is selected from polysaccharides, proteins and any combinations or copolymers thereof; preferably is selected from collagen, chitosan, alginate and any combinations or copolymers thereof. In one embodiment, the matrix comprises collagen. In one embodiment, the matrix consists in collagen. In another embodiment, the matrix is made of a mixture of collagen and chitosan, or a mixture of collagen and alginate. In one embodiment, the biocompatible and/or resorbable polymer is a mixture of at least one endogen polymer and at least one exogen polymer. In one embodiment, the biocompatible and/or resorbable polymer is not or does not comprise any proteins secreted by Engelbreth-Holm-Swarm (EHS) mouse sarcoma cells, preferably the biocompatible and/or resorbable polymer is not or does not comprise the product Matrigel® manufactured by Corning Life Sciences.

In one embodiment, the three-dimensional matrix is porous.

In one embodiment, the three-dimensional matrix a hydrogel. Preferably, the three-dimensional matrix a porous hydrogel. In one embodiment, the three-dimensional matrix results from a hydrogel, preferably results from drying or freeze-drying a hydrogel.

The present invention thus provides a biomaterial comprising:

- a three-dimensional matrix made of at least one biocompatible and/or resorbable polymer, and
- particles encapsulating a complement component, a complement active fragment, or a combination thereof; said particles being dispersed in the three-dimensional matrix,
- wherein said particles are polymeric microspheres and the polymer of the microspheres is selected from polycaprolactone (PCL), poly(lactic acid) (PLA), poly(glycolic acid) (PGA), polyhydroxybutyrate (PHB), poly(3-hydroxy valerate), poly(ethylene succinate) (PESu), poly(butylene succinate) (PBSu) and any combinations or copolymers thereof.

In one embodiment, the biomaterial of the invention comprises polymeric microspheres encapsulating complement active fragment C5a, said microspheres being dispersed in the three-dimensional matrix. In one embodiment, the biomaterial of the invention comprises PLGA microspheres encapsulating a complement component, a complement active fragment, or a combination thereof; said microspheres being dispersed in the three-dimensional matrix. In one embodiment, the biomaterial of the invention comprises polymeric microspheres encapsulating a complement component, a complement active fragment, or a combination thereof; said microspheres being dispersed in a collagen three-dimensional matrix. In one embodiment, the biomaterial of the invention comprises PLGA microspheres encapsulating complement active fragment C5a, said microspheres being dispersed in the three-dimensional matrix. In one embodiment, the biomaterial of the invention comprises PLGA microspheres encapsulating a complement component, a complement active fragment, or a combination thereof; said microspheres being dispersed in a collagen three-dimensional matrix. In one embodiment, the biomaterial of the invention comprises polymeric microspheres encapsulating complement active fragment C5a, said microspheres being dispersed in a collagen three-dimensional matrix. In one embodiment, the biomaterial of the invention comprises PLGA microspheres encapsulating complement active fragment C5a, said microspheres being dispersed in a collagen three-dimensional matrix.

In one embodiment, the encapsulating particles are randomly dispersed in the three-dimensional matrix. In one embodiment, the encapsulating particles are homogeneously dispersed in the three-dimensional matrix. In one embodiment, the encapsulating particles are randomly dispersed in the pores of three-dimensional matrix. In one embodiment, the encapsulating particles are homogeneously dispersed in the pores of the three-dimensional matrix.

In a specific embodiment, the biomaterial of the invention is biphasic. This means that the biomaterial comprises:

- a first phase as described above, i.e. a three-dimensional matrix made of at least one biocompatible and/or resorbable polymer in which particles encapsulating a complement component, a complement active fragment, or a combination thereof, are dispersed; and
- a second phase, which is free of complement component or complement active fragment, said second phase being for example a three-dimensional matrix made of at least one biocompatible and/or resorbable polymer ;

said first and second phases being in contact together.

In one embodiment, the second phase is a three-dimensional matrix made of at least one biocompatible and/or resorbable polymer which is free of complement component or complement active fragment, especially free of particles encapsulating a complement component or complement active fragment. In a preferred embodiment, the polymer of the matrix of the second phase is the same as the one used to form the matrix of the complement component/complement active fragment-loaded phase.

Advantageously, a dye is added in one of the two phases, preferably in the first phase (loaded phase), in order to be able to identify the loaded part of the biphasic biomaterial. For example, the dye may be patent blue V dye. The presence of a dye is important to correctly orientate the material in the lesion to be treated.

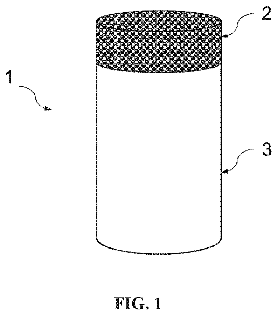

A biphasic biomaterial is illustrated in Figure 1. A cylindrical sample (1) of a biphasic biomaterial is represented, comprising a first phase (2) which is a complement component/complement active fragment-loaded matrix and a second phase (3) which is an unloaded matrix, both phases being in contact together.

Preferably the two phases of the biphasic biomaterial are made of the same polymer and there is thus a continuity in the matrix.

In one embodiment, the ratio of the volume of the first phase (loaded-phase) to the volume of the second phase (unloaded phase) is ranging from 0.01 to 1; preferably from 0.05 to 0.5; preferably from 0.1 to 0.25. According to one embodiment, the ratio of the volume of the first phase (loaded-phase) to the volume of the second phase (unloaded phase) is ranging from 0.01 to 0.9; preferably from 0.01 to 0.8; from 0.01 to 0.7; from 0.01 to 0.6; from 0.01 to 0.5; from 0.01 to 0.4 or from 0.01 to 0.3. According to one embodiment, the ratio of the volume of the first phase (loaded-phase) to the volume of the second phase (unloaded phase) is about 0.01; 0.02; 0.03; 0.04; 0.05; 0.06; 0.07; 0.08 or 0.09.

In the case of a biphasic cylindrical biomaterial for use in dental restauration, the ratio of the thickness of the first phase (loaded-phase) to the thickness of the second phase (unloaded phase) is ranging from 0.01 to 1, preferably from 0.03 to 0.7; preferably from 0.07 to 0.5. According to one embodiment, the ratio of the thickness of the first phase (loaded-phase) to the thickness of the second phase (unloaded phase) is ranging from 0.01 to 0.9; preferably from 0.01 to 0.8; from 0.01 to 0.7; from 0.01 to 0.6; or from 0.01 to 0.5. According to one embodiment, the ratio of the thickness of the first phase (loaded-phase) to the thickness of the second phase (unloaded phase) is about 0.01; 0.02; 0.03; 0.04; 0.05; 0.06; 0.07; 0.08; 0.09; 0.1: 0.2; 0.3; 0.4; 0.5; 0.6 or 0.7.

In one embodiment, the biomaterial of the invention further comprises additional bioactive agents. The bioactive agents may be for example biological materials such as cytokines, growth factors, peptides, antibodies, antibiotics or differentiating factors. The bioactive agents may also include therapeutic agents.

In one embodiment, the biomaterial of the invention does not comprise cells, especially does not comprise stem cells. In an alternative embodiment, the biomaterial of the invention comprises cells, especially stem cells.

In one embodiment, the biomaterial has a loading rate of complement component and/or complement active fragment ranging from 0.001% to 0.1%; preferably from 0.005% to 0.05%; more preferably is about 0.01%, in weight to the total weight of the biomaterial. According to one embodiment, the biomaterial has a loading rate of complement component and/or complement active fragment ranging from 0.001% to 0.1%; preferably from 0.001% to 0.09%; 0.001% to 0.08%; 0.001% to 0.07%; 0.001% to 0.06%; 0.001% to 0.05%; 0.001% to 0.04%; 0.001% to 0.03%; 0.001% to 0.02% or 0.001% to 0.01%.

In one embodiment, the biomaterial of the invention has a size and volume adapted to the lesion to be repaired. In one embodiment, the biomaterial of the invention has preferably a volume ranging from 0.01 to 160 cm$^3$. In the case of dental tissue regeneration, the biomaterial of the invention has preferably a volume ranging from 10 to 100 mm$^3$, preferably from 15 to 50 mm$^3$, preferably from 30 to 40 mm$^3$.

In one embodiment, the biomaterial of the invention has a shape selected from cylinder, parallelepiped, cube. In one embodiment, the biomaterial of the invention has a shape suitable to fill a natural or artificial cavity or chamber of a tooth.

In one embodiment, the biomaterial of the invention is porous. Such a porous structure provides space for cell migration, adhesion and growth of regenerated tissues. In one embodiment, the biomaterial has pores having a diameter ranging from 1 $\mu$m to 1500 $\mu$m, preferably from 100 $\mu$m to 500 $\mu$m, preferably from 200 $\mu$m to 400 $\mu$m. According to one embodiment, the biomaterial has pores having a diameter ranging from 1 $\mu$m to 1400 $\mu$m; preferably from 1 $\mu$m to 1300 $\mu$m; from 1 $\mu$m to 1200 $\mu$m; from 1 $\mu$m to 1100 $\mu$m; from 1 $\mu$m to 1000 $\mu$m; from 1 $\mu$m to 900 $\mu$m; from 1 $\mu$m to 800 $\mu$m; from 1 $\mu$m to 700 $\mu$m; from 1 $\mu$m to 600 $\mu$m; from 1 $\mu$m to 500 $\mu$m; or from 1 $\mu$m to 400 $\mu$m. According to one embodiment, the biomaterial has pores having a diameter of about 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 or 400 $\mu$m.

In one embodiment, the biomaterial of the invention is delivered for example by injection, insertion, or implantation at the target tissue.

In some embodiments, the biomaterial is delivered to a natural or artificial cavity or chamber of a tooth of a subject by injection, especially when the biomaterial is under the form of a hydrogel.

## Manufacturing of the biomaterial

The manufacturing of the biomaterial of the invention can be performed by methods known by skilled artisan. The present invention also provides methods of manufacturing of said biomaterial.

In one embodiment, the manufacturing of the biomaterial of the invention comprises contacting particles encapsulating a complement component and/or complement active fragment with at least one biocompatible and/or resorbable polymer to form a three-dimensional matrix made of said polymer in which the particles are dispersed.

In one embodiment, the encapsulation of the complement component, complement active fragment, or combination thereof, is performed using a water/oil single emulsion method or a water/oil/water double emulsion method. In a preferred embodiment, encapsulation is performed using a water/oil/water double emulsion method.

In one embodiment, encapsulation under the form of polymeric microspheres performed by a water/oil/water double emulsion method comprises the following steps:

a) preparing:

- an oily phase comprising the encapsulating polymer and an organic solvent; and
- a first aqueous phase comprising the complement component, complement active fragment, or combination thereof, and water;

b) emulsifying the oily phase and the first aqueous phase to form a water-in-oil emulsion;
c) emulsifying the water-in-oil emulsion obtained in step b) with a second aqueous phase to form a water-in-oil-in-water double emulsion;
d) removing the organic solvent by extraction in water;
e) recovering formed polymeric microspheres.

In one embodiment, the organic solvent is selected from dichloromethane, dimethylsulfoxide (DMSO) and acetone; preferably the organic solvent is selected from dichloromethane and dimethylsulfoxide (DMSO); more preferably is dichloromethane.

In one embodiment, the first aqueous phase comprising the complement component and/or complement active fragment further comprises bovine serum albumin (BSA). The presence of BSA presents the advantage to avoid the adsorption of the complement component and/or complement active fragment to the wall of the containers used during the encapsulation process. Moreover, BSA also enables to stabilize the emulsion during the encapsulation process.

In one embodiment, the second aqueous phase comprises a surfactant such as for example polyvinyl alcohol (PVA), polyoxyethylene (100) stearyl ether, poly(ethylene glycol)-block-poly(propylene glycol)-block-poly(ethylene glycol) average Mn ~2,900, polysorbate 20, polysorbate 60, polysorbate 80, Span 20, Span 60, Span 80. In one embodiment, the second aqueous phase comprises a surfactant selected from PVA and Span 20; preferably PVA. Advantageously, the amount of PVA in the second aqueous phase is ranging from 0.01% to 0.5% in weight to the total volume of the second aqueous phase (w/v); preferably from 0.05% to 0.15% w/v; more preferably about 0.1 % w/v.

In one embodiment, emulsification is performed by sheer homogenization, ultrasonication, microfluidization; preferably by sheer homogenization. Preferably, emulsification is performed using a high-speed mixing apparatus.

In one embodiment, the manufacturing of the polymeric microspheres is performed at room temperature.

In one embodiment, step d) of removing of the organic solvent is performed by pouring the double emulsion into a large volume of water; preferably under stirring.

In one embodiment, step e) of recovering of formed polymeric microspheres is performed by centrifugation and/or washing with water.

Advantageously, isolated polymeric microspheres are lyophilized and stored at -20°C.

In one embodiment, the manufacturing of the biomaterial of the invention comprises contacting particles encapsulating a complement component, complement active fragment, or combination thereof, with a hydrogel formed with the biocompatible and/or resorbable polymer.

In one embodiment, the hydrogel formed with the biocompatible and/or resorbable polymer is prepared by dissolution of the polymer in an aqueous solution followed by homogenization. In one embodiment, the amount of polymer in the hydrogel is ranging from 0.1% to 5% in weight of the total weight of the hydrogel (w/w); preferably from 0.5% to 2%; more preferably from 1% to 1.5%. In one embodiment, the aqueous solution in which the polymer is dissolved to form the hydrogel may be water (preferably purified water) or an acid water solution such as a 0.25% w/w; acetic acid aqueous solution. Preferably, when the polymer is collagen or alginate, the dissolution is performed in water; and when the polymer is chitosan, dissolution is performed in acid water solution. In one embodiment, homogenization is performed by mixing, preferably at a rate ranging from 100 to 20000 rpm, preferably from 300 to 15000 rpm. In one embodiment, homogenization is performed for a period of time ranging from 5 min to 2 hours, preferably from 10 minutes to 1 hour. Especially, homogenization of collagen hydrogel is performed at a speed of 14000 rpm for a period of time of about 10 minutes; and homogenization of chitosan or alginate hydrogels is performed at a speed of 300 rpm for a period of time of about 1 hour. In case wherein the matrix is made of a mixture of polymers (for example chitosan/collagen or alginate/collagen), each gel is prepared individually and then mixed; preferably mixed for 30 min at 300 rpm before loading with the particles encapsulating the complement active fragment and/or complement component.

The loading of the hydrogel of biocompatible and/or resorbable polymer with particles encapsulating the complement active fragment and/or complement component is performed by mixing the particles with the hydrogel; preferably by magnetic stirring at 500 rpm, preferably for about 30 minutes. In one embodiment, the loaded amount of encapsulating particles is ranging from 1 to 50 mg of particles per mL of gel; preferably from 2 to 30 mg of particles per mL of gel, more preferably about 4 mg of particles per mL of gel or about 30 mg of particles per mL of gel.

The loaded hydrogel can either be directly used as such or it can undergo a further step of water removal.

Water removal enables for example to provide the biomaterial under the form of a sponge. In one embodiment, water removal can be performed by freeze-drying. Preferably, the hydrogel is first distributed in a mold of shape of interest and then freeze-dried. The design of the mold should be adapted to the size requirement of the expected sponges. The mold can be manufactured for example by 3D printing.

In the case of biphasic biomaterial preparation, a first hydrogel loaded with particles is prepared as described above, preferably with a concentration of particles of 30 mg of microspheres per mL of gel. A second hydrogel, free of particles encapsulating complement components and/or complement active fragments, is also prepared in the same manner. Advantageously, a dye is added in one of the two hydrogels in order to be able to identify the loaded part of the biphasic biomaterial. For example, the dye may be patent blue V dye. The presence of a dye is important to correctly orientate the material in the lesion to be treated. Then, the first hydrogel (loaded gel) is distributed in a mold to partially fill said mold and then the second hydrogel (unloaded gel) is distributed to complete the filling of the mold. Freeze-drying then enable to obtained a biphasic sponge.

**Uses**

The biomaterial of the invention is useful for tissue repair and regeneration, especially with regards to dental tissues or bone tissues, or other tissues such as nervous tissues and skin tissues. Therefore, in one embodiment, it is provided a biomaterial according to the invention for use in tissue repair and/or regeneration.

The invention thus provides a biomaterial of the invention for use in the treatment of tissue repair and/or regeneration by contacting the tissue to be repaired and/or regenerated with the biomaterial of the invention. By "contacting the tissue" is intended to include any form of interaction (e.g., direct or indirect interaction) between the biomaterial and the tissue to be repaired and/or regenerated. Contacting the tissue with the biomaterial may be performed either *in vivo* or *in vitro.* In certain embodiments, the tissue is contacted with the biomaterial *in vitro* and subsequently transferred into a subject in an *ex vivo* method of administration. Contacting the tissue with the biomaterial *in vivo* may be done, for example, by placing the biomaterial into the tissue, or by injecting the biomaterial into another area adjacent to the tissue, such that cells within the tissue will travel towards the biomaterial.

In one embodiment, the dental tissue is selected from dental pulp, dentin, periodontal and gingival tissues. In one embodiment, the biomaterial according to the invention is for use in repairing and/or regenerating dental pulp, dentin, periodontal and/or gingival tissues.

In one embodiment, the dental tissue is contacted with the biomaterial of the invention by placing the biomaterial into a natural or artificial cavity or chamber of a tooth to be treated.

The biomaterial of the invention is particularly useful in dentin-pulp regeneration. For such use, the biomaterial may be used under the form of a hydrogel or of a sponge. The use of biphasic biomaterial as described above, preferably a biphasic sponge, is particularly advantageous for dentin-pulp regeneration because the complement active fragment(s) diffusion provides a gradient for directing the migration of stem cells to the site where dentin regeneration is necessary/required.

Especially, the biomaterial of the invention can be used in direct pulp capping or in pulpotomy procedures. The use of the biomaterial of the invention enables to obtain a complete regeneration of the pulp volume. It enables to avoid an important reduction of the pulp volume, contrary to what is observed with previous materials used in such treatments. This is particularly advantageous because reduction of the pulp volume leads to a reduced pulp-dentin regeneration capacity. This is particularly true in case of secondary caries/tooth fracture.

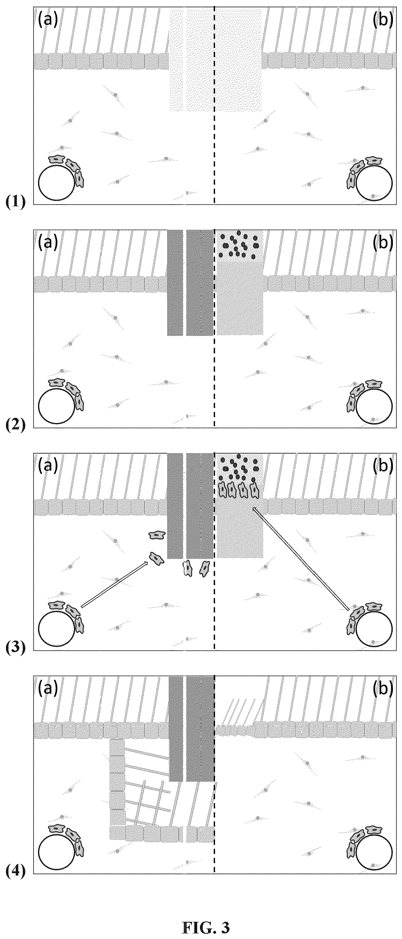

The mechanism of dentin-pulp regeneration is represented in Figure 3 for a direct pulp capping or pulpotomy procedure, comparing the use of the biomaterial of the invention under biphasic form (right side (b)) and the use of a classic direct pulp-capping material (left side (a)). Figure 3(1) shows a damaged tooth with a carious lesion, wherein a part of dentin and pulp were removed in order to proceed to a direct pulp capping or pulpotomy. Figure 3(2) shows the placement of a material filling the cavity, respectively a classic direct pulp-capping material (a) or the biphasic biomaterial of the invention (b) with the loaded phase on the upper part, i.e. the loaded phase is in the upper part of the dentin, while the unloaded phase extends from the bottom of the dentin into the pulp. Figure 3(3) shows the migration of dental pulp stem cells. With a classic direct pulp-capping material, cells migrate at the interface thereof, while with the biphasic biomaterial of the invention, cells colonize the biomaterial, especially colonize the unloaded phase up to the loaded phase. Consequently, in the case of a classic direct pulp-capping material, regeneration of dentin occurs at the interface with the filling material leading to a reduction of the pulp volume, as represented on Figure 3(4)-(a). Conversely, with the biphasic biomaterial of the invention, pulp is regenerated as well as dentin, with regeneration of dentin close to the physiological dentin level, leading to a complete regeneration of the pulp volume, as represented on Figure 3(4)-(b).

The invention thus provides a biomaterial of the invention for use in the treatment of a periodontal disease, i.e. a dental pulp infection, in a subject in need thereof. The use of the biomaterial of the invention includes contacting dental pulp with the biomaterial of the invention. Periodontal diseases generally range from simple gum inflammation to serious disease that results in major damage to the soft tissue and bone that support the teeth.

The invention thus provides a biomaterial of the invention for use in the treatment of a dental pulp infection in a subject in need thereof. The use of the biomaterial of the invention includes contacting dental pulp with the biomaterial of the invention. Dental infections may arise from pulpitis, i.e., inflammation of the dental pulp tissue, which begins on the tooth's surface as dental caries. Dental infections may result in an exposure of pulp tissue, and eventually lead to a decay and/or loss of dentin and/or pulp, which are conventionally treated by endodontic surgery, e.g., root canal therapy.

A subject in need of the therapeutic uses described herein can be a subject having, diagnosed with, suspected of having, or at risk for developing an injury, disease or condition associated with dental tissue, such as dental trauma, pulpitis or dental caries. For example, a subject in need of the therapeutic uses described herein can be a subject having, diagnosed with, suspected of having, or at risk for developing reversible pulpitis or other diseases or conditions that conventionally require removal of healthy dental pulp along with diseased pulp. A determination of the need for treatment will typically be assessed by a history and physical exam consistent with the disease or condition at issue. Diagnosis of the various conditions treatable by the methods described herein is within the skill of the art. The subject can be an animal subject, including a mammal, preferably a human subject.

In one embodiment, the biomaterial of the invention is used to perform a dental, endodontic or root canal procedure on a mammalian tooth in need thereof. The method can include exposing traumatized or diseased dental pulp tissue in the tooth pulp chamber and/or root canal; and capping or filling at least a portion of the tooth pulp chamber and/or root canal with the biomaterial of the invention. In one embodiment, the biomaterial does not include living cells during the capping or filling. In another embodiment, the biomaterial includes living cells. In one embodiment, the method further comprises removing traumatized or diseased dental pulp tissue from the tooth to create a tooth pulp chamber and/or root canal substantially devoid of traumatized or diseased tissue.

When used in direct pulp capping or in pulpotomy procedures, the material of the invention is placed in the cavity to be regenerated and may then be covered by a sealing material. The sealing material may be for example a tricalcium silicate based cement such as for example Biodentine (Septodont), a mineral trioxide aggregate (MTA). When used in periodontal guided tissue regeneration procedures, the material of the invention can be covered by a membrane.

The biomaterial of the invention is also useful in bone repair and/or regeneration, with applications in orthopedics and implantology.

In one embodiment, the biomaterial according to the invention is thus for use in repairing and/or regenerating bone tissues.

In this type of use, using a monophasic biomaterial is particularly preferred because a homogenous colonization of the biomaterial by stem cells is required to regenerate the missing tissue.

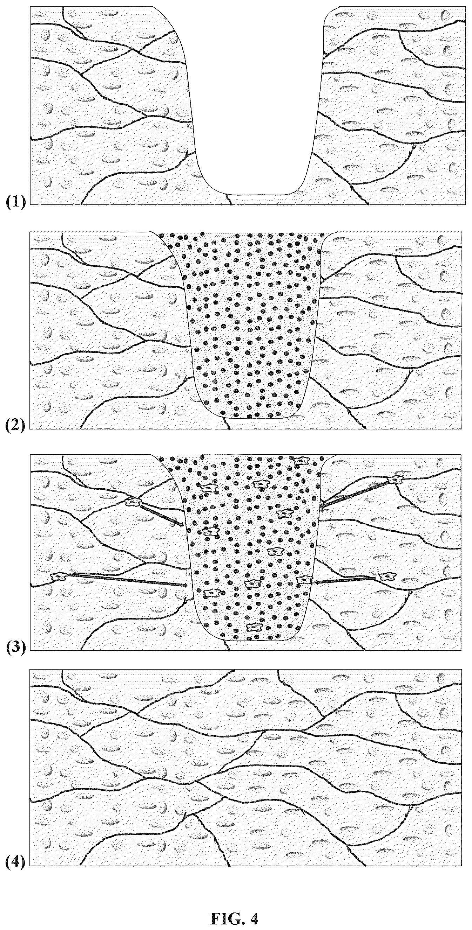

The mechanism of bone regeneration, using a monophasic biomaterial of the invention, is represented in Figure 4. Figure 4(1) shows a bone tissue with a bone defect.

Figure 4(2) shows the placement of a monophasic biomaterial of the invention filling the cavity. Figure 4(3) shows the migration of bone progenitor cells which colonize the biomaterial, enabling to regenerate the bone tissues as represented on Figure 4(4).

The biomaterial of the invention is also useful in nervous tissues repair and/or regeneration. In one embodiment, the biomaterial according to the invention is thus for use in repairing and/or regenerating nervous tissues. Nervous tissues include neurons and neuroglial cells (including astrocytes, microglial cells, ependymal cells, oligodendrocytes, satellite cells and Schwann cells).

The biomaterial of the invention is also useful in skin repair and/or regeneration. In one embodiment, the biomaterial according to the invention is thus for use in repairing and/or regenerating skin. Skin tissues include epidermis, dermis and hypodermis.

In such applications, the biomaterial is preferably used under the form of a solid framework. Especially, the biomaterial of the invention may be inserted in a solid scaffold, for example in a solid scaffold made of resorbable tissue replacement (RTR) granules made of tricalcium phosphate, or of hydroxyapatite, or a material partially resorbable, comprising silicates and having an open porosity. This present the advantage of allowing its colonization by stem cells.

In one embodiment, the biomaterial of the invention comprises C5a as complement active fragment. In such case, the biomaterial enables promoting recruitment of progenitor cells of pulp. The invention thus provides a method for recruiting of progenitor cells of pulp comprising contacting dental pulp with the biomaterial of the invention.

In one embodiment, the biomaterial of the invention is suitable a cell culture substrate. This is particularly advantageous for bone regeneration.

**BRIEF DESCRIPTION OF THE DRAWINGS**

**Figure 1** is a perspective view of a cylindrical biphasic biomaterial of the invention.

**Figure 2** is a series of photographs showing monophasic (a) and biphasic (b) C5a-microspheres-loaded collagen matrices according to the invention and a magnification (c) showing microspheres dispersed in the matrix.

**Figure 3** is a series of schemes representing the dentin-pulp regeneration process in a direct pulp capping or pulpotomy procedure. On the left side (a) of schemes (1) to (4) is represented a classic direct pulp capping or pulpotomy procedure, while on the right side (b) is represented a direct pulp capping or pulpotomy procedure using the biomaterial of the invention under biphasic form. Scheme (1) represents a damaged tooth with a carious lesion, wherein a part of dentin and pulp are removed. Scheme (2) shows the placement of a material filling the cavity in dentin and pulp: (a) a classic direct pulp-capping material and (b) the biphasic biomaterial of the invention, with the loaded phase on the upper part. Scheme (3) shows the migration of dental pulp stem cells. Scheme (4) shows the final state of the tooth after the direct pulp capping or pulpotomy procedure.

**Figure 4** is a series of schemes representing the bone regeneration process. Figure 4(1) shows a bone tissue with a bone defect. Figure 4(2) shows the placement of a monophasic biomaterial of the invention filling the cavity. Figure 4(3) shows the migration of bone progenitor cells which colonize the biomaterial, enabling to regenerate the bone tissues as represented on Figure 4(4).

**Figure 5** is a series of 3 photographs (a), (b) and (c), showing PLGA microspheres encapsulating C5a complement active fragment. The indicated scale is 10.00 $\mu$m.

**Figure 6** is a graph representing the cumulated percentage of C5a release overtime from microspheres encapsulating C5a placed in DMEM.

**Figures 7a** and **7b** are graphs showing the dental pulp fibroblasts viability overtime for different materials. The control is the DMEM medium. Collagen sponges were tested, either unloaded collagen sponges or C5a-encapsulating-microspheres loaded collagen sponges, either monophasic or biphasic. Figure 7a reports the results of the direct evaluation, while Figure 7b reports the results of the indirect evaluation.

**Figure 8** is a graph representing the migration overtime of dental pulp fibroblasts in presence of the biomaterial of the invention.

**Figure 9** is a graph representing the C5a release overtime from a collagen sponge loaded with C5a encapsulated in microspheres.

**Figure 10** is a graph representing the C5a release overtime from a collagen sponge loaded with free C5a.

## EXAMPLES

The present invention is further illustrated by the following examples.

### Abbreviations

BSA: bovine serum albumin,
DCM: dichloromethane,
DMSO: dimethylsulfoxide,
h: hour,
min: minute,
PLGA: poly(lactic-co-glycolic acid);
PVA: polyvinyl alcohol,
rpm: round per minute.

### Material

C5a (R&D system, France); Kit ELISA C5a (R&D system, France). DMEM (Dulbecco's Modified Eagle's Medium) and all cell culture materials and reagents: Fischer Scientific (PAA laboratories) - France. Magnetic beads: Dynal Biotech, Oslo - Norway. Primary antibodies: R&D Systems, Lille - France. Collagen (Fibrillar collagen provided by Datascope, Getinge Group, USA), Chitosan (Sigma), Alginate (Septodont, France), PLGA (EXPANSORB® DLG 50-2A provided by PCAS, France), PVA (Merck, Germany).

### I. PARTICLES ENCAPSULATING COMPLEMENT ACTIVE FRAGMENTS

#### 1.1. Manufacturing of polymer microspheres encapsulating C5a

Microspheres of PLGA were prepared by a water/oil/water (w/o/w) double-emulsion and solvent extraction/evaporation method adapted from the method previously described by Kalaji et al. (Kalaji N. et al., J. Biomed. Nanotech., 2010, 6(2), pp. 1-11). In brief, PLGA was dissolved in DCM to form the oil phase. This oil phase was then emulsified using a high-speed mixing apparatus (Ultrathurax®, T25 basic, IKA® Werke/Germany) with an internal aqueous phase of purified water containing C5a always with BSA to form a w/o emulsion. All preparations were performed at room temperature. The resulting emulsion was added to 50 mL of external aqueous solution containing 0.1% (w/v) PVA and emulsified with Ultrathurax® in order to produce the double w/o/w emulsion. The double emulsion was then poured into a large volume of water (100 mL) under magnetic stirring for 2 hours to allow removal of the organic solvent. Finally, the resulting microspheres were centrifuged, washed twice with 50 mL of deionized water, lyophilized and stored at -20°C.

#### I.2. Characterization of polymer microspheres encapsulating C5a

Microspheres' morphology was analyzed using a Keyence VHX-5000 digital microscope. This device allows to automatically reconstruct a sharp and deep image, thanks to its intuitive software making measurements of microspheres size in a very short time.

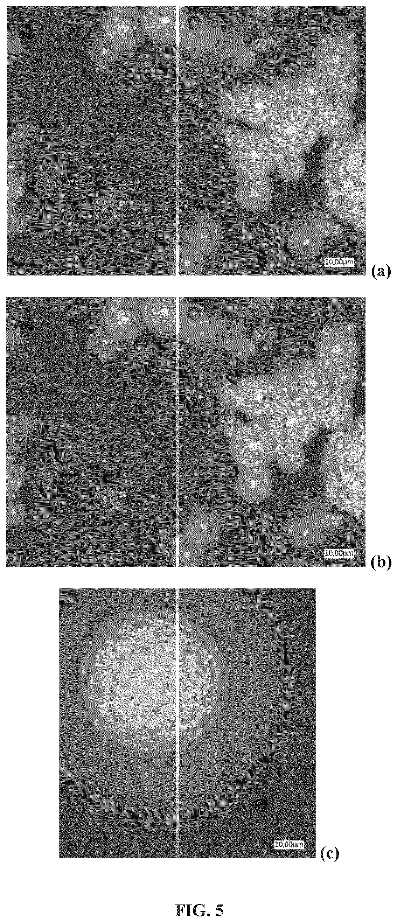

The particles obtained by the w/o/w double-emulsion and solvent extraction/evaporation method described above in example I.1. are under the form of microspheres. The outer surface of the microspheres has a rough aspect, probably reflecting on their porosity, as illustrated in Figure 5. The microspheres have mean size of 25.51 (+ 9.83) $\mu$m in

diameter.

I.3. Encapsulation efficiency

The encapsulation efficiency test aims at determining the quantity complement component and/or complement active fragment (for example C5a) present in the particles.

This is particularly important to adjust the quantity of complement component and/or complement active fragment in the biomaterial.

It is important that the encapsulation efficiency is high in order to limit the required amount of particles in the biomaterial in order to avoid the presence of a too important amount of PLGA which might be toxic.

Two methods were used to measure the encapsulation efficiency:

- Method 1 (direct determination) consists in measuring the amount of complement active fragment entrapped in the microspheres after extraction. The extraction protocol consists in dissolving about 5 mg of microspheres in 1 mL of DMSO, then adding 9 mL of DMEM (Dulbecco's modified eagle medium) and analyzing by ELISA the quantity of complement active fragment trapped in the microspheres (described below in section III.2).
- Method 2 (indirect determination) comprises determining the lost quantity of complement active fragment in the aqueous supernatant at the end of the microspheres preparation (after solvent evaporation, including rinsing water) using an ELISA quantification. The lost quantity is compared to the initial amount of complement active fragment involved in the encapsulation process to determine the encapsulated amount.

The encapsulation efficiency of the microspheres encapsulating C5a obtained in example I.1 was determined. The two methods, direct and indirect, are concordant. The encapsulation efficiency varies from 70% to 79%, with an average of 74.79 (+ 3.72) %.

I.4. Liberation assay

The liberation assay aims at determining the amount of complement component and/or complement active fragment (for example C5a) that can be released from the encapsulating microspheres.

Polymeric microspheres encapsulating C5a were placed in a fixed volume of DMEM at 37°C, 5% $CO_2$. At 1, 7, 14, 21 and 31 days, the medium is removed and stored at -20°C and fresh medium is added. At each time point a dosage of liberated C5a is performed using ELISA assay (described in section III.2).

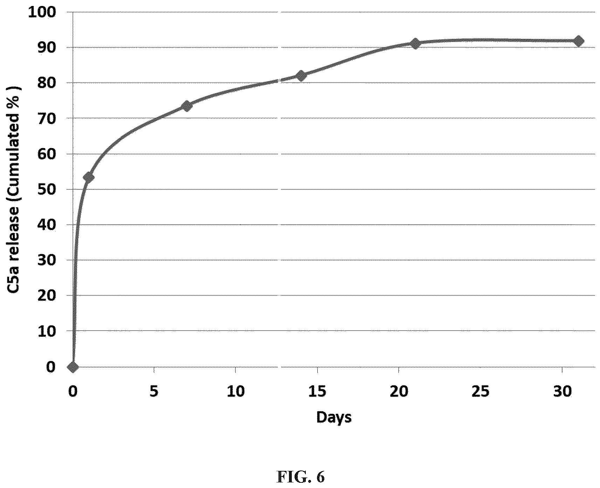

The liberation of C5a from the microspheres encapsulating C5a obtained in example 1.1 was analyzed. As shown in Figure 6, an important burst release of C5a was observed the first day of the liberation assay. During this burst release, 53% of the C5a present in the microspheres is released. This initial burst release was followed by a slow release of C5a during 21 days where 91% of the encapsulated C5a has been released.

II. BIOMATERIAL OF THE INVENTION: MANUFACTURING AND CHARACTERIZATION

II.1. Manufacturing of unloaded matrices

Porous solid matrices were prepared using a freeze-drying method. First a hydrogel was prepared by dissolution of the chosen biocompatible and/or resorbable polymer (for example collagen, chitosan or alginate - see table 1 below) in an aqueous solution and homogenized. The gel was then distributed in a mold to fit the size requirement (3 mm in diameter and 5 mm in height). The gel was then frozen for 12 h at -40°C and freeze dried using the following parameters: sublimation at -40°C, 12 mbar, during 24h, to provide a matrix under the form of a sponge.

Table 1: Composition of the gel used for matrix preparation.

| Polymer | Amount in the gel (w/w) | Dissolution | Homogenization | |
| --- | --- | --- | --- | --- |
| | | | Speed | Duration |
| **Collagen (fibers)** | 1.5 % | Purified water | 14000 rpm | 10 min |
| **Chitosan (powder)** | 1 % | Acetic acid 0.25% (w/w) aqueous solution | 300 rpm | 1h |
| **Alginate (powder)** | 1 % | Purified water | 300 rpm | 1h |

In the case of a mixed matrix (for example chitosan 1%/collagen 1% or alginate 1%/collagen 1%), each gel is prepared individually and then mixed for 30 min at 300 rpm before distribution.

II.2. Manufacturing of matrices loaded with C5a-microspheres

Microspheres-loaded porous solid matrices were prepared as described in example II.1, with a further step of loading of the gel with polymer microspheres encapsulating C5a of example 1.1 (4 mg of microspheres per mL of gel) before distribution in the molds. After 30 min magnetic stirring at 500 rpm, the gel is distributed in the mold, frozen and lyophilized using the parameters previously described, to provide a loaded matrix under the form of a sponge.

In the case of biphasic porous solid matrix preparation (i.e. a material comprising a first phase of microspheres-loaded matrix and a second phase of unloaded matrix), the microspheres-loaded gel is prepared in the same way, with a concentration of microspheres of 30 mg of microspheres per mL of gel and addition of 0.5 mg of patent blue V dye was added. The microspheres-loaded gel is first distributed in the mold and then the unloaded gel is distributed to complete the filling of the mold. Freeze-drying then enables obtaining a biphasic sponge. In one example, 10 $\mu$l of loaded gel were distributed in the mold, followed by 60 $\mu$l of unloaded gel.

For comparative purposes, matrices loaded with free C5a were also prepared. Free C5a-loaded collagen sponges were prepared as described in example II.1, with a further step of loading of the gel with free C5a (4 $\mu$g of C5a per mL of gel) before distribution in the molds. After 30 min magnetic stirring at 500 rpm, to allow an even distribution of C5a in the collagen gel, the gel is distributed in the mold, frozen and lyophilized using the parameters previously described, to provide a free C5a-loaded collagen sponge.

II.3. Loading rate of C5a in the three-dimensional matrix

The determination of the loading rate of C5a in the biomaterial enable to know the amount of C5a present in the biomaterial.

For matrices loaded with free C5a, an indirect approach was used to determine the C5a loading rate: the C5a lost during the freeze drying and in the foils used for transportation was measured using ELISA assay. This amount of C5a lost during the manufacturing process was deduced from the amount of C5a engaged in the experiment (4$\mu$g/mL of gel), enabling to calculate the loading rate.

*Results.* For the free C5a loaded scaffold obtained in example II.2, it was measured that 1 $\mu$g of C5a was lost for 1 mL of collagen gel. The loading rate in C5a of the material is thus of 75% in weight of the total weight of C5a used in the biomaterial manufacturing process.

For the microspheres loaded matrices, an indirect approach was also used to determine the C5a loading rate: in this case, the microspheres are trapped in the collagenic matrix and cannot be lost during transportation or freeze drying. Using the encapsulation efficiency calculated for the microspheres, it is possible to calculate the amount of C5a loaded in the matrix using the concentration of microspheres loaded in the gel.

*Results.* For the microspheres loaded matrices, the encapsulation efficiency of the microspheres is of 75% (average), as determined in example I.3. The microspheres are dispersed in the gel, and the C5a is not lost during this step. Therefore, the loading rate in C5a of the material is thus of 100% in weight of the total weight of C5a used in the biomaterial manufacturing processes and of 75% is the encapsulation step is also considered.

II.4. Biomaterial morphology characterization by digital microscopy

Porous solid matrices' morphology was analyzed using the Keyence VHX-5000 digital microscope.

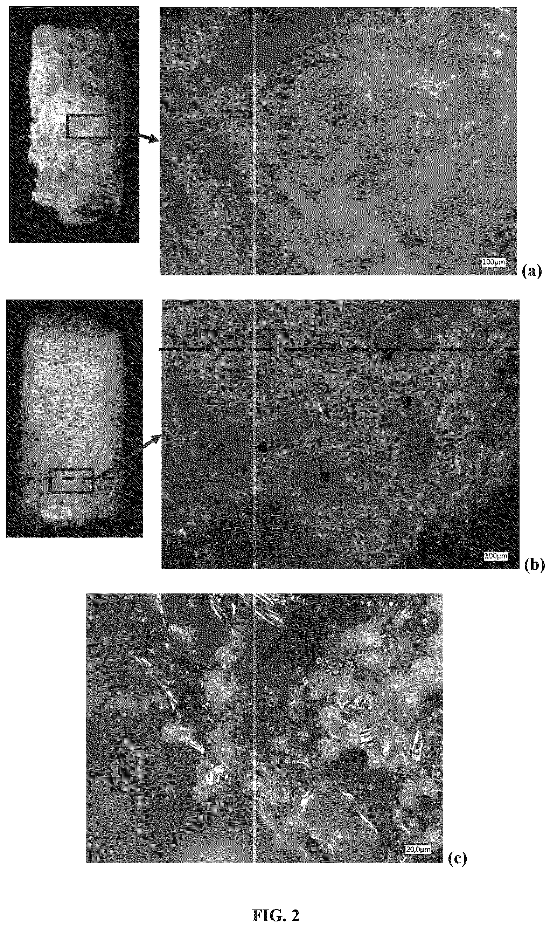

The monophasic and biphasic microspheres-loaded matrices obtained in example II.2 were analyzed and the morphology of the loaded matrices can be seen in Figure 2. Especially, Figure 2(a) shows a monophasic microspheres-loaded collagen sponge obtained as described in example II.2; and a magnification (scale 100 $\mu$m) of the sponge structure. Figure 2(b) shows a biphasic microspheres-loaded collagen sponge obtained as described in example II.2. The dotted line shows the demarcation between the loaded phase at the bottom and the unloaded phase on the top. The triangles in the magnification (scale 100 $\mu$m) on the right point some of the encapsulated microspheres.

Figure 2(c) is a further magnification (scale 20.0 $\mu$m) of encapsulated microspheres dispersed in a collagen sponge matrix.

II.5. Water uptake assay - Absorption factor

The water uptake test aims at simulating the blood absorption and assessing the water uptake by the porous network. The test is inspired from Hui-Min Wang works on collagen/hyaluronic acid/gelatin sponges (Hui-Min Wang et al., PLOS ONE, 2013, 8(6), e56330).

More specifically, the dry material is weighted before being plunged into a beaker of fluid. Once the fluid spontaneously penetrates the matrix, bubbles of water are removed by pressing the material. This step is critical since air bubbles constitute an unused volume and can influence slightly the water intake. The material is then removed from the fluid with pin tweezers. At this step, the material must not be damaged or squeezed. Water is allowed to drain from the material, without squeezing. Once no more drops form, the wet material is weighted.

Absorption factor calculated using the formula below:

$$\text{Absorption factor} = \frac{\text{wet material weight} - \text{dry material weight}}{\text{dry material weight}}$$

*Results*

The absorption factor allows the comparison of the water uptake of the different biomaterials. The porosity of the biomaterial influences the absorption factor calculated using the formula above. The biomaterial comprising matrices of collagen 1.5%, collagen 1% - chitosan 1% and collagen 1% - alginate 1% have similar absorption factor.

The absorption factor of unloaded sponges obtained as described in example II.1 was determined as explained above (table 2):

Table 2: Absorption factor of unloaded sponges.

| 3D matrix | Absorption factor |
|---|---|
| Collagen 1.5% | 33 |
| Collagen 1% - Chitosan 1% | 35 |
| Collagen 1% - Alginate 1% | 33 |

These results show that all tested sponges have a similar absorption of water. This is important since the sponge needs to absorb the blood in order to allow the migration of the cells necessary for the regeneration.

II.6. Degradation assay

The degradation test is inspired from Davidenko et, al. works (Davidenko et al., Acta Biomaterialia, 2015, 25, pp. 131-142) and aims at determining the degradation of the material in water at 37°C.

For each tested material, 4 sponges of known masses are placed on a beaker in 20 mL of water at 37°C in order to monitor their dissolution as a function of the incubation time by quantifying their mass loss. At each time $t_x$ of monitoring, the sample is taken from the dissolution medium, freeze-dried and weighted. The mass loss is calculated as follows:

$$\%\text{mass loss} = \frac{\text{dry sponge mass (at t0)} - \text{dry sponge mass (at tx)}}{\text{dry sponge mass (at t0)}} * 100$$

The total duration of the test is defined depending on the sample state in the beaker, according to whether it is still present or already totally degraded.

*Results*

Unloaded sponges obtained as described in example II.1 were tested. The collagen sponge has a slow degradation rate, and following the degradation assay, it was shown that after 14 days, only 33% of the scaffold is lost. The mixed sponges (collagen - chitosan or collagen - alginate) adopt the degradation rate of the collagen.

PART III. BIOLOGICAL EVALUATION

III.1. Cells preparation and characterization

**Collection of Molar Teeth.** Human immature third molars freshly extracted for orthodontic reasons and carious teeth were obtained in compliance with French legislation (including informed consent and institutional review board approval of the protocol used).

**Primary Pulp Cell Culture.** Human pulp cells were prepared from the immature third molars at the two-thirds root formation stage by the explant outgrowth method. Briefly, pulp is extracted from the teeth. The pulp is then mechanically cut to form small pulp explants. Each explant is placed in a small volume of DMEM in a petri dish. After 24h, the petri dish is filled with medium. Cells outgrowing of the explants are collected constituting a pulp cell population.

**Magnetic Cell Sorting.** Pulp progenitor cells were directly sorted from primary pulp cell cultures at passages 1 to 5 with mouse anti-human STRO-1 IgM with immune magnetic beads according to the manufacturer's protocol (Dynal, Oslo, Norway). Briefly, magnetic beads are coated with a mouse anti human STRO-1 IgM. Cells are then added on the prepared beads. Once the STRO-1 antigen is fixed to its specific antibody, the cell populations are separated using the beads. The non-fixed population, or negative fraction, is a pulp fibroblasts population. The fixed population, or positive fraction, is a pulp stem cell population. On this latter population, beads need to be washed once the cells adhere to the culture flask.

**Pulp Cell Characterization.** This test is used to confirm what cells are present after magnetic cell sorting. STRO-1 sorted and non-sorted pulp cells were grown in 8-well glass culture chambers up to 70% confluence. In these 2 cultures, cells were rinsed with phosphate buffered saline (PBS) and fixed with 4% paraformaldehyde during 15 min at 4°C. for 1 h. While non-sorted pulp cells were incubated for 1 h with primary antibody against fibroblast surface protein (FSP; 2 $\mu$g/mL), STRO-1 sorted cells were incubated with primary antibodies against STRO-1 (5 $\mu$g/mL) and CD44 (2.5 $\mu$g/mL), CD90 (2.5 $\mu$g/mL), CD105 (2.5 $\mu$g/mL), CD146 (2.5 $\mu$g/mL), or CD166 (2.5 $\mu$g/mL). Controls were incubated with the respective primary antibody isotypes. After washing, cells were incubated for 45 min with their respective secondary antibody, Alexa Fluor 488 or Alexa Fluor 594 (2 $\mu$g/mL), and with DAPI (1 $\mu$g/mL) for fluorescence microscopy detection.

III.2. C5a concentration determined by ELISA assay

C5a concentration is determined by an Enzyme-Linked ImmunoSorbent Assay (ELISA) according to the manufacturer's instructions (R&D Systems, Lille, France). Briefly, a 96 wells plate was coated with a primary antibody against C5a protein over-night at 4°C. After 1h in a blocking solution, the samples are placed in the wells and incubated for 2h at room temperature. A biotinylated secondary antibody against C5a is then added for 2h at room temperature. Streptavidin coupled with HRP enzyme will then be added to the wells. The revelation occurs by the addition of TMB that is transformed by the HRP enzyme into a blue colored substrate. Plate are read at 650nm wavelength.

III.3. Toxicity assay

Toxicity of the biomaterial was assayed *in vitro* using dental pulp fibroblasts (STRO-1 negative fraction of dental pulp cells after magnetic sorting).

Cells are seeded in 96 wells plate and culture to subconfluency. Media containing biomaterials' eluates, were prepared by incubating samples in DMEM for 24h. Fibroblasts were incubated with conditioned media for 24 and 72h (indirect method). The biomaterial has also been placed directly in contact with the cells (direct method). At each time point a MTT test was performed. MTT is placed on the cells, at a concentration of 1 mg/mL and incubated for 2h, allowing the formation of formazan crystal. After 2h incubation, the crystals are dissolved using DMSO. The plate is read at 550 nm wavelength.

*Results.* The cell viability with the different materials of the invention has been evaluated. The control was the DMEM medium alone and gave a cell viability was 100%. The toxicity of the encapsulating microspheres was evaluated with the MTT test.

The results showed that the encapsulating microspheres (whether loaded with C5a or unloaded) were not toxic to the cells.

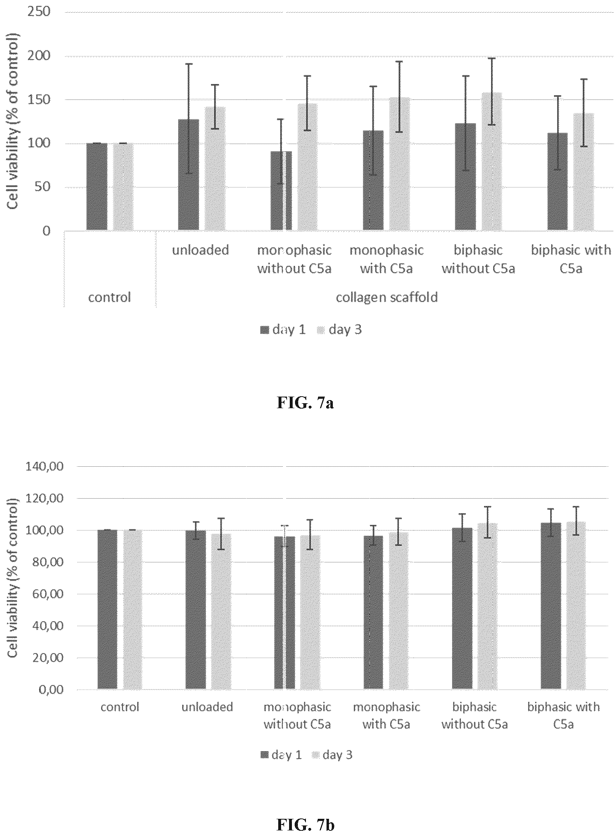

The toxicity of the unloaded collagenic matrix and of monophasic or biphasic C5a-encapsulating-microspheres-loaded collagen sponges was also evaluated directly and indirectly with the MTT test, as presented in the graphs on Figures 7a and 7b, respectively. The results showed that there is no toxicity of the loaded or unloaded collagen matrices to the dental pulp cells.

Chitosan, collagen - chitosan and collagen - alginate matrices, loaded and unloaded, have also been tested via indirect toxicity assay. The results showed no toxicity of these matrices.

III.4. Migration assay

To assess the activity of the C5a released by the biomaterial, a migration assay was performed. Indeed, the C5a protein is known for its role in mobilizing immune and stem cells and this function is the most important one for the regeneration of the dentin-pulp complex.

Migration was assayed in 24-well plates equipped with transwell inserts with an 8-$\mu$m pore-size membrane. STRO-1 sorted cells ($10^4$ cells/well) were resuspended in serum-free MEM and seeded in the upper chamber. The lower chambers were loaded with conditioned media. After 24 h, non-migrating STRO-1 sorted cells on the upper side of the insert membrane were wiped off using a cotton bud, and STRO-1 sorted cells on the lower surface of the same membrane were fixed with ethanol and stained with hematoxylin. The number of migrating STRO-1 sorted cells to the lower surface of the membrane was counted in 5 random fields under a light microscope (×200).

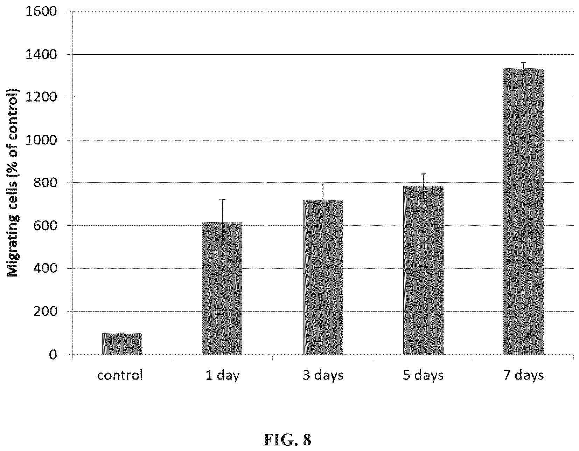

*Results.* In the control condition of this experiment (DMEM medium), only few dental pulp stem cells migrated. The presence of C5a released from the biomaterial of the invention significantly increased the migration of dental pulp stem cells until day 7 as reported in Figure 8.

III.5. C5a liberation assay

An encapsulated C5a-loaded matrix is placed in a fixed volume of DMEM at 37°C, 5% $CO_2$. At 1, 7, 14, 21 and 31 days, the medium is removed and stored at -20°C and fresh medium is added. At each time point a dosage of liberated C5a is performed using ELISA assay (described in section III.2).

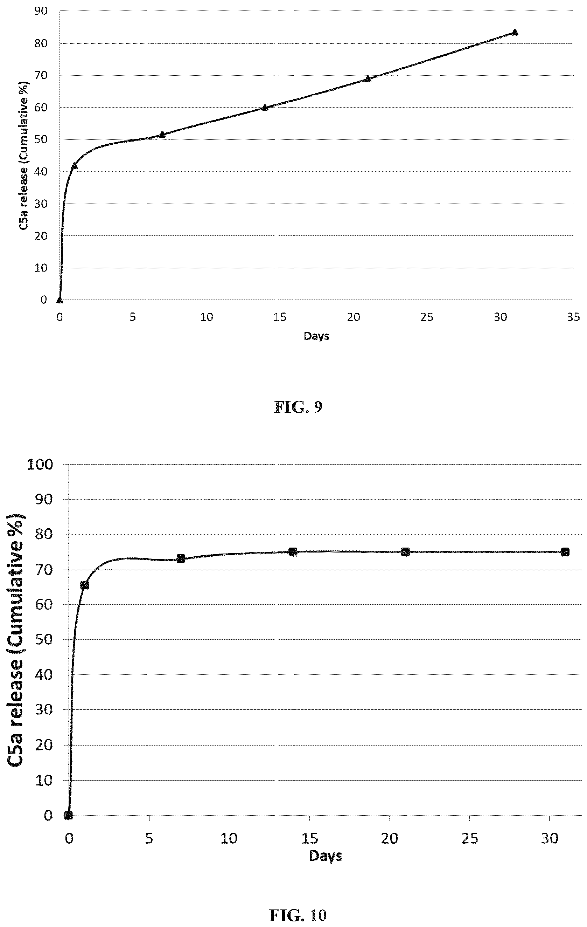

*Results.* As showed on Figure 9, the incorporation of C5a-encapsulating-microspheres in collagen matrix allows a slow and controlled release of C5a in the conditions of the test. On the first day, an important burst release of C5a is observed. During this burst release, 42% of the C5a present in the biomaterial is release. After this initial burst release, a slow and controlled release of C5a was obtained until 31 days. Results are expressed in percentage of C5a present in the scaffold.

*Comparative example - free C5a-loaded matrix.* A collagen sponge loaded with free C5a (example II.2) was tested in the same conditions of liberation assay. It was observed that 90% of the C5a is liberated at 1 day in a high burst release (Figure 10). After this initial burst release, no more C5a is released from the matrix. In the conditions of the assay, the liberated C5a remained stable and active for 7 days. Nevertheless, it is known that C5a has a short half-life in vivo, namely of less than 1 hour.

**Claims**

1. A biomaterial comprising:

   - a three-dimensional matrix made of at least one biocompatible and/or resorbable polymer, and
   - particles encapsulating a complement component, a complement active fragment, or a combination thereof; said particles being dispersed in the three-dimensional matrix;
   - wherein said particles are polymeric microspheres and the polymer of the microspheres is selected from polycaprolactone (PCL), poly(lactic acid) (PLA), poly(glycolic acid) (PGA), polyhydroxybutyrate (PHB), poly(3-hydroxy valerate), poly(ethylene succinate) (PESu), poly(butylene succinate) (PBSu) and any combinations or copolymers thereof.

2. The biomaterial according to claim **1,** wherein the biocompatible and/or resorbable polymer is selected from polysaccharides, proteins and any combinations or copolymers thereof; preferably is selected from collagen, chitosan, alginate and any combinations or copolymers thereof; more preferably is collagen.

3. The biomaterial according to claim **1** or claim **2,** wherein the polymer microspheres are made of poly(lactic-co-glycolic acid) (PLGA).

4. The biomaterial according to any one of claims **1** to **3,** wherein the complement component and complement active fragment are selected from complement component C1, complement component C2, complement component C3, complement component C4, complement component C5, complement active fragments thereof such as C5a or C3a and any combinations thereof; preferably complement component C5 or C3; more preferably complement active fragment C5a or C3a.

5. The biomaterial according to any one of claims **1** to **4,** comprising PLGA microspheres encapsulating complement active fragment C5a, said microspheres being dispersed in a collagen three-dimensional matrix.

6. The biomaterial according to any one of claims **1** to **5,** further comprising a second phase made of a three-dimensional matrix made of at least one biocompatible and/or resorbable polymer which is free of particles encapsulating a complement component, a complement active fragment, or a combination thereof.

7. The biomaterial according to any one of claims **1** to **6,** wherein the three-dimensional matrix is porous.

8. The biomaterial according to any one of claims **1** to **7,** wherein the three-dimensional matrix is a hydrogel.

9. A process for manufacturing the biomaterial according to any one of claims **1** to **8,** said process comprising contacting particles encapsulating a complement component, a complement active fragment, or a combination thereof, with at least one biocompatible and/or resorbable polymer to form a three-dimensional matrix made of the at least one biocompatible and/or resorbable polymer in which the particles are dispersed; wherein said particles are polymeric microspheres and the polymer of the microspheres is selected from polycaprolactone (PCL), poly(lactic acid) (PLA), poly(glycolic acid) (PGA), polyhydroxybutyrate (PHB), poly(3-hydroxy valerate), poly(ethylene succinate) (PESu), poly(butylene succinate) (PBSu) and any combinations or copolymers thereof.

10. The process according to claim **9,** wherein the particles encapsulating a complement component, a complement active fragment, or a combination thereof, are contacted with the biocompatible and/or resorbable polymer by impregnation in a hydrogel prepared by dissolution of said polymer in an aqueous solution followed by homogenization.

11. The process according to claim **10,** further comprising a step of water removal to provide the biomaterial under the form of a sponge.

12. A biomaterial according to any one of claims **1** to **8,** for use in tissue repair and/or regeneration.

13. The biomaterial for use according to claim **12,** wherein the tissue is selected from dental pulp, dentin, periodontal and gingival tissues.

14. The biomaterial for use according to claim **12,** wherein the tissue is selected from bones, nervous tissues and skin tissues.

**Patentansprüche**

1. Biomaterial, das Folgendes umfasst:

   - eine dreidimensionale Matrix aus mindestens einem biokompatiblen und/oder resorbierbaren Polymer, und
   - Partikel, die eine Komplementkomponente, ein aktives Komplementfragment oder eine Kombination davon einkapseln; wobei die Partikel in der dreidimensionalen Matrix dispergiert sind;
   - wobei die Partikel polymere Mikrosphären sind und das Polymer der Mikrosphären ausgewählt ist aus Polycaprolacton (PCL), Polymilchsäure (PLA), Polyglykolsäure (PGA), Polyhydroxybutyrat (PHB), Poly(3-hydroxyvalerat), Poly(ethylensuccinat) (PESu), Poly(butylensuccinat) (PBSu) und beliebige Kombinationen oder Copolymeren davon.

2. Biomaterial nach Anspruch **1,** wobei das biokompatible und/oder resorbierbare Polymer aus Polysacchariden, Proteinen und deren Kombinationen oder Copolymeren ausgewählt ist; bevorzugt aus Kollagen, Chitosan, Alginat und beliebigen Kombinationen oder Copolymeren davon ausgewählt ist und noch bevorzugter Kollagen ist.

3. Biomaterial nach Anspruch **1** oder Anspruch **2,** wobei die Polymermikrosphären aus Poly(milchsäure-co-glykolsäure) (PLGA) bestehen.

4. Biomaterial nach einem der Ansprüche **1** bis **3,** wobei die Komplementkomponente und das aktive Komplementfragment aus der Komplementkomponente C1, der Komplementkomponente C2, der Komplementkomponente C3, der Komplementkomponente C4, der Komplementkomponente C5, den aktiven Komplementfragmenten davon wie beispielsweise C5a oder C3a und beliebigen Kombinationen davon; bevorzugt der Komplementkomponente C5 oder C3 und noch bevorzugter dem aktiven Komplementfragment C5a oder C3a ausgewählt sind.

5. Biomaterial nach einem der Ansprüche **1** bis **4,** umfassend PLGA-Mikrosphären, die das aktive Komplementfragment C5a einkapseln, wobei die Mikrosphären in einer dreidimensionalen Kollagenmatrix dispergiert sind.

6. Biomaterial nach einem der Ansprüche **1** bis **5,** das ferner eine zweite Phase aus einer dreidimensionalen Matrix aus mindestens einem biokompatiblen und/oder resorbierbaren Polymer umfasst, das frei von Partikeln ist, die eine Komplementkomponente, ein aktives Komplementfragment oder eine Kombination davon einkapseln.

7. Biomaterial nach einem der Ansprüche **1** bis **6,** wobei die dreidimensionale Matrix porös ist.

8. Biomaterial nach einem der Ansprüche **1** bis **7,** wobei die dreidimensionale Matrix ein Hydrogel ist.

9. Verfahren zur Herstellung des Biomaterials nach einem der Ansprüche **1** bis **8,** wobei das Verfahren das Inkontaktbringen von Partikeln, die eine Komplementkomponente, ein aktives Komplementfragment oder eine Kombination davon einkapseln, mit mindestens einem biokompatiblen und/oder resorbierbaren Polymer umfasst, um eine dreidimensionale Matrix aus dem mindestens einen biokompatiblen und/oder resorbierbaren Polymer zu bilden, in der die Partikel dispergiert sind;
wobei die Partikel polymere Mikrosphären sind und das Polymer der Mikrosphären ausgewählt ist aus Polycaprolacton (PCL), Polymilchsäure (PLA), Polyglykolsäure (PGA), Polyhydroxybutyrat (PHB), Poly(3-hydroxyvalerat), Poly(ethylensuccinat) (PESu), Poly(butylensuccinat) (PBSu) und beliebige Kombinationen oder Copolymeren davon.

10. Verfahren nach Anspruch **9,** wobei die Partikel, die eine Komplementkomponente, ein aktives Komplementfragment oder eine Kombination davon einkapseln, mit dem biokompatiblen und/oder resorbierbaren Polymer durch Imprägnierung in einem Hydrogel, das durch Auflösung des Polymers in einer wässrigen Lösung und anschließender Homogenisierung hergestellt wird, in Kontakt gebracht werden.

11. Verfahren nach Anspruch **10,** das ferner einen Schritt zur Wasserentfernung umfasst, um das Biomaterial in Form eines Schwamms bereitzustellen.

12. Biomaterial nach einem der Ansprüche **1** bis **8,** zur Anwendung bei der Gewebereparatur und/oder -regeneration.

13. Biomaterial zur Verwendung nach Anspruch **12,** wobei das Gewebe aus Zahnpulpa, Dentin, Parodontal- und Gingivagewebe ausgewählt ist.

14. Biomaterial zur Verwendung nach Anspruch **12,** wobei das Gewebe aus Knochen, Nervengewebe und Hautgewebe ausgewählt ist.

**Revendications**

1. Biomatériau comprenant :

- une matrice tridimensionnelle constituée d'au moins un polymère biocompatible et/ou résorbable, et
- des particules encapsulant un composant du complément, un fragment actif du complément ou une combinaison de ceux-ci ; lesdites particules étant dispersées dans la matrice tridimensionnelle ;
- dans lequel lesdites particules sont des microsphères polymériques et le polymère des microsphères est choisi parmi le polycaprolactone (PCL), le poly(acide lactique) (PLA), le poly(acide glycolique) (PGA), le polyhydroxybutyrate (PHB), le poly(3-hydroxyvalérate), le poly(éthylène succinate) (PESu), le poly(butylène succinate) (PBSu) et toute combinaison ou copolymère de ceux-ci.

2. Biomatériau selon la revendication **1,** dans lequel le polymère biocompatible et/ou résorbable est choisi parmi les polysaccharides, les protéines et toute combinaison ou copolymère de ceux-ci ; préférentiellement est choisi parmi le collagène, le chitosane, l'alginate et toute combinaison ou copolymère de ceux-ci ; plus préférentiellement est le collagène.

3. Biomatériau selon la revendication **1** ou la revendication **2,** dans lequel les microsphères polymères sont constituées de poly(acide lactique-co-glycolique) (PLGA).

4. Biomatériau selon l'une quelconque des revendications **1** à **3,** dans lequel le composant du complément et le fragment actif du complément sont choisis parmi le composant Cl du complément, le composant C2 du complément, le composant C3 du complément, le composant C4 du complément, le composant C5 du complément, leurs fragments actifs tels que C5a ou C3a et toute combinaison de ceux-ci ; préférentiellement le composant C5 ou C3 du complément ; plus préférentiellement le fragment actif C5a ou C3a du complément.

**5.** Biomatériau selon l'une quelconque des revendications **1** à **4,** comprenant des microsphères de PLGA encapsulant le fragment actif du complément C5a, lesdites microsphères étant dispersées dans une matrice tridimensionnelle de collagène.

**6.** Biomatériau selon l'une quelconque des revendications **1** à **5,** comprenant en outre une deuxième phase constituée d'une matrice tridimensionnelle constituée d'au moins un polymère biocompatible et/ou résorbable exempt de particules encapsulant un composant du complément, un fragment actif du complément, ou une combinaison de ceux-ci.

**7.** Biomatériau selon l'une quelconque des revendications **1** à **6,** dans lequel la matrice tridimensionnelle est poreuse.

**8.** Biomatériau selon l'une quelconque des revendications **1** à **7,** dans lequel la matrice tridimensionnelle est un hydrogel.

**9.** Procédé de fabrication du biomatériau selon l'une quelconque des revendications **1** à **8,** ledit procédé comprenant la mise en contact de particules encapsulant un composant du complément, un fragment actif du complément, ou une combinaison de ceux-ci, avec au moins un polymère biocompatible et/ou résorbable afin de former une matrice tridimensionnelle constituée dudit au moins un polymère biocompatible et/ou résorbable dans laquelle les particules sont dispersées ;
dans lequel lesdites particules sont des microsphères polymériques et le polymère des microsphères est choisi parmi le polycaprolactone (PCL), le poly(acide lactique) (PLA), le poly(acide glycolique) (PGA), le polyhydroxybutyrate (PHB), le poly(3-hydroxyvalérate), le poly(éthylène succinate) (PESu), le poly (butylène succinate) (PBSu) et toute combinaison ou copolymère de ceux-ci.

**10.** Procédé selon la revendication **9,** dans lequel les particules encapsulant un composant du complément, un fragment actif du complément, ou une combinaison de ceux-ci, sont mises en contact avec le polymère biocompatible et/ou résorbable par imprégnation dans un hydrogel préparé par dissolution dudit polymère dans une solution aqueuse suivie d'une homogénéisation.

**11.** Procédé selon la revendication **10,** comprenant en outre une étape d'élimination de l'eau pour fournir le biomatériau sous la forme d'une éponge.

**12.** Biomatériau selon l'une quelconque des revendications **1** à **8,** pour utilisation dans la réparation et/ou la régénération tissulaire.

**13.** Biomatériau pour utilisation selon la revendication **12,** dans lequel le tissu est choisi parmi la pulpe dentaire, la dentine, les tissus parodontaux et gingivaux.

**14.** Biomatériau pour utilisation selon la revendication **12,** dans lequel le tissu est choisi parmi les os, les tissus nerveux et les tissus cutanés.

FIG. 1

FIG. 2

**FIG. 3**

**FIG. 4**

FIG. 5

FIG. 6

**FIG. 7a**

**FIG. 7b**

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013098331 A

**Non-patent literature cited in the description**

- **CHMILEWSKY F. et al.** *J. Dent. Res.*, 2013, vol. 92 (6), 532-539
- **IGNATIUS A. et al.** *J. Trauma.*, 2011, vol. 71, 952-960
- **MANTHEY et al.** *Int. J. Biochem. Cell Biol.*, 2009, vol. 41, 2114-2117
- **KALAJI N. et al.** *J. Biomed. Nanotech.*, 2010, vol. 6 (2), 1-11
- **DAVIDENKO et al.** *Acta Biomaterialia*, 2015, vol. 25, 131-142